# EUROPEAN PATENT APPLICATION

(11) **EP 4 439 040 A2**
(43) Date of publication of application: **02.10.2024**
(21) Application number: 24186595.5
(22) Date of filing: 05.11.2018
(51) Int. Cl.: G01N 1/38

(54) **HEPARIN-BASED BLOOD SAMPLER WITHOUT PLATELET ACTIVATION**

(30) Priority: 15.11.2017 DK PA201700652
(62) Divisional of application: 18796664.3
(71) Applicant: Radiometer Medical ApS, 2700 Brønshøj (DK)
(72) Inventor: BURKHARDT, Melanie Andrea, 2700 Brønshøj (DK)
(74) Representative: Inspicos P/S

(57) **Abstract**

The present invention relates to blood sampler and the preparation of blood samples that can be used for not only for blood gas, basic metabolic panel parameter analysis but also for a platelet count and/or white blood count, such as a 3-diff or 5-diff. The blood samples comprise at least one anticoagulant for the determination of blood gas and basic metabolic panel parameters and at least one anti-platelet agent.

## Description

### Technical Field

The present invention relates to the field of diagnostic blood sample analysis.

### Background of the invention

Rapid access to blood tests is a mainstay in the diagnosis and treatment of acute disease. Oxygenation status and acid-base balance are determined by arterial blood gas (BG) analysis and constitute a central part of modern evidence-based treatment algorithms in critical care. Furthermore, devices intended for critical care testing allow for assessment of e.g. electrolytes, renal function (creatinine), inflammation (C-reactive protein) and cardiac biomarkers.

The basic metabolic panel (BMP) is used to check the status of a person's kidneys and their electrolyte and acid/base balance, as well as their blood glucose level - all of which are related to a person's metabolism. It can also be used to monitor hospitalized patients and people with certain known conditions, such as hypertension and hypokalemia.

Further, the count of white blood cells (WBC) is an important biomarker for several diseases and a differential WBC count may even differentiate five different types of blood cells ("5-diff" or "5-part diff"), namely neutrophils, lymphocytes, monocytes, eosinophils and basophils. Alternatively, WBC can be differentiated into granulocytes (neutrophils, basophils and eosinophils reported as a group), lymphocytes and monocytes ("3-diff" or "3-part diff"). Each is reported as a percentage. A shift in the percentage may indicate a pathological condition.

Further, platelets (also termed thrombocytes) may be counted as another parameter. Platelets are small fragments of cells that are essential for normal blood clotting. A platelet count may be used to screen for or diagnose various diseases and conditions that can cause problems with clot formation. It may be used as part of the workup of a bleeding disorder, bone marrow disease, or excessive clotting disorder, to name just a few.

The test may be used as a monitoring tool for people with underlying conditions or undergoing treatment with drugs known to affect platelets. It may also be used to monitor those being treated for a platelet disorder to determine if therapy is effective.

However, blood samples usually have to be differently prepared for diagnostic measurements of the abovementioned parameters. For example, for BG and BMP parameter analysis, the standard anticoagulant is heparin. Heparin prevents blood coagulation, but it however does not prevent platelet (thrombocyte) activation and aggregation, which leads to platelet aggregate formation. Heparin is therefore nowadays not used for a complete blood cell count (CBC) analysis, comprising the counts of WBC, platelets, 3-diff or 5-diff, red blood cell (RBC) concentration, hematocrit, hemoglobin concentration and RBC descriptive parameters. The measured platelet count in heparinized blood would be underestimated, in particular when using state of the art automated hematology analyzers, which are not able to distinguish single platelets from aggregated platelet clots. Instead, platelet aggregates may be misclassified by hematological analyzers as leucocytes and therefore, a falsely high WBC count is obtained potentially resulting in a flawed diagnosis or flags and error messages rendering the results unusable.

Ethylenediaminetetraacetic acid (EDTA), either as di-sodium, di-potassium or tripotassium salt, is another commonly used standard anticoagulant in hematology. The use of EDTA is generally accepted to be safe and reliable for obtaining complete blood cell counts. In addition, EDTA salts are compatible, i.e. do not interfere, with standard staining protocols for blood smears. If problems with EDTA-dependent pseudothrombocytopenia occur, citrate is used as the alternative anticoagulant. However, EDTA or citrate cannot be used for BG and BMP parameter analysis as these anticoagulants strongly interfere with electrolyte measurements. For example, EDTA and citrate form a complex with Ca²⁺ and thus interfere with Ca²⁺ measurements and these anticoagulants might even destroy the calcium-sensors of the automated analyzers.

Currently, hematology analysis, and in particular CBC, is performed on EDTA or citrate anticoagulated blood samples and not on heparinized blood samples. Consequently, a comprehensive analysis of CBC, BG and BMP parameters so far needs to be performed with separate differently anticoagulated blood samples on separate instruments.

Schnuff-Wemet et al. (Br. J. Haematol. 162, 684, 2013) describe that MgSO₄ may be used as anticoagulant for blood samples of patients with pseudothrombocytopenia as an alternative to EDTA or citrate.

US 2010/0280412 discloses blood coagulation factor Xa inhibitors and a method for anticoagulation of human blood in which blood calcium concentration remains the same and no thrombin is formed and thrombocyte function is not affected.

US 6,880,384 B2 describes an automated blood analyzer for measuring blood gas parameters, metabolic parameters and electrolytes as well as a blood sampler containing the blood sample and a stirring element.

In the light of the above, there is a need to provide a single blood sample that can be used for BG, BMP, platelet and WBC count to allow that all parameters can be determined using the same automated blood analyzer and the same blood sample. This would reduce the patient's burden that several blood samples need to be drawn for determining the various blood parameters described above.

It is thus an object of the present invention to provide methods for preparing a blood sample for blood analysis that may be used for a "3-in-1" analysis of BG, BMP and platelet count and optionally a WBC (or CBC) count.

It is a further object of the present invention to provide suitable means for preparing said blood sample for blood analysis that may be used for a "3-in-1" analysis of BG, BMP and platelet count and optionally a WBC (or CBC) count.

### Summary

The objects are solved by an *in vitro* method for preparing a blood sample, wherein blood is combined with
a) at least one anticoagulant for the determination of blood gas and basic metabolic panel parameters; and
b) at least one anti-platelet agent.

The objects are also solved by a method for preparing a blood analysis sample comprising the step of
a) drawing blood from a patient into a blood sampler;
wherein the blood sampler contains at least one anticoagulant for the determination of BG and BMP parameter analysis and at least one anti-platelet agent.

Further, the objects are solved by an *in vitro* method for determining blood gas and BMP parameter and platelet count in a blood sample comprising the step of
a) determining BG, BMP parameter and/or number of platelets in a blood sample obtained by described above.

Moreover, the present invention relates to the use of at least one anticoagulant for the determination of BG and BMP parameters and at least one anti-platelet agent for the preparation of a blood sample for BG and BMP parameter analysis and platelet count, preferably in a blood sampler.

In another aspect, the objects are solved by the use of a blood sampler comprising blood, at least one anticoagulant for the determination of BG and BMP parameter analysis and at least one anti-platelet agent for the analysis of BG, BMP parameter and/or platelet count.

Moreover, the objects are solved by a blood sampler containing
a) at least one anti-coagulant agent for BG and BMP parameter analysis; and
b) at least one anti-platelet agent.

### Detailed description

The present invention relates to an *in vitro* method for preparing a blood sample, wherein blood is combined with a) at least one anticoagulant for the determination of BG and BMP parameters; and b) at least one anti-platelet agent. It was surprisingly found that the blood sample prepared according to the inventive method is suitable for BG and BMP parameter analysis as well as platelet count. It is thus one advantage of the present invention inventive method that it allows a "3-in-1" analysis with one blood sample. In a preferred embodiment, the blood sample is additionally suitable for WBC count. The blood sample obtained by the inventive method is thus preferably suitable for BG and BMP parameter analysis as well as complete blood cell count (CBC).

In another aspect, the present invention relates to a method for preparing a blood analysis sample comprising the step of
a) drawing blood from a patient into a blood sampler;
wherein the blood sampler contains at least one anticoagulant for the determination of blood gas and BMP parameter analysis and at least one anti-platelet agent. The blood analysis sample preferably comprises relatively low (i.e. small) volumes of blood, e.g. compared to blood samples obtained for a blood donation, which can be about 450 mL blood. In a preferred embodiment, the blood sample drawn from the patient contains from about 20 µL to about 10 mL, preferably from about 35 µL to about 10 mL, more preferably from 50 µL to about 5 mL and even more preferably from about 0.5 to about 2 mL.

Further, the present invention also relates to an *in vitro* method for determining blood gas and BMP parameter and platelet count in a blood sample comprising the step of
a) determining BG, BMP parameter and/or number of platelets in a blood sample obtained by the methods described above. One advantage of the inventive method is that all parameters described above can be measured from one sample. In a preferred embodiment, the method further comprises a step of determining the WBC count.

In a preferred embodiment, the determination of BG and BMP parameters as well as the platelet count is performed with an automated blood analyzer. Suitable blood analyzers are for example described in US 5,564,419 or US 6,880,384.

The blood sample is typically whole blood. The blood may either be venous blood or arterial blood. It is preferred that the blood is arterial blood. However, the use of venous blood may be preferred when the inventive method is used for preparing blood samples in the setting of an emergency department.

In one embodiment, the blood may be capillary blood. This embodiment is especially preferred if the blood sample is obtained from neonates.

In a preferred embodiment, the blood of the blood sample is not a purified fraction of a specific type of blood cells, e.g. a population of washed platelets.

In a preferred embodiment of the above inventive methods, the at least one anticoagulant for the determination of BG and BMP parameters is selected from the group consisting of indirect factor Xa inhibitors or direct factor Xa inhibitors or combinations thereof.

In a preferred embodiment, the anticoagulant is selected from the group of heparinates or heparinoids or combinations thereof. Preferred embodiments of heparinates are heparin, such as unfractionated, high molecular weight heparin (HMWH), low molecular weight heparin (LMWH) including bemiparin, certoparin, dalteparin, enoxaparin, nadroparin, parnaparin, reviparin, tinzaparin; and oligosaccharides such as fondaparinux, idraparinux. Preferred embodiments of heparinoids comprise danaparoid, dermatan sulfate and sulodexide.

Preferred embodiments of direct factor Xa inhibitors comprise apixaban, betrixaban, darexaban, edoxaban, otamixaban and rivaroxaban.

In a preferred embodiment, the at least one anticoagulant suitable for BG and BMP parameter analysis comprises heparin. As described above, heparin is the standard anticoagulant for BG and BMP parameter analysis. Heparin is a naturally occurring polysaccharide that inhibits coagulation, the process that leads to thrombosis. Natural heparin consists of molecular chains of varying lengths, or molecular weights. It is also used as an anticoagulant medication (blood thinner). It binds to the enzyme inhibitor antithrombin III (AT), causing a conformational change that results in its activation through an increase in the flexibility of its reactive site loop. The activated AT then inactivates thrombin, factor Xa and other proteases.

In a preferred embodiment, the anticoagulant is electrolyte-balanced heparin, (also called "balanced heparin"). Heparin is known to bind positively charged electrolytes and this may interfere with electrolyte measurements. It is preferred that the formulations of electrolyte-balanced heparin comprise lithium, zinc, sodium, potassium or ammonium salts of heparin. In a preferred embodiment, the formulation of electrolyte-balanced heparin comprises lithium heparin and sodium heparin.

In another embodiment, the anticoagulant heparin is human heparin, pig heparin or synthetic heparin. In a preferred embodiment, the anticoagulant is pig heparin.

In another preferred embodiment, the anticoagulant is unfractionated heparin.

In a preferred embodiment, the final concentration of the anticoagulant, e.g. heparin, is from about 10 IU/mL to about 200 IU/mL, preferably from about 20 IU/mL to about 100 IU/mL. In a particularly preferred embodiment, the final concentration of the anticoagulant, .e.g. heparin, is about 60 IU/mL.

In one embodiment, the at least one anticoagulant for the determination of BG and BMP parameters used according to the present invention can be in a liquid form, also called "liquid heparin", or in a dry form, e.g. as dry balanced heparin when combined with the blood. One example of a dry form of the at least one anticoagulant is a lyophilized anticoagulant, e.g. lyophilized heparin or lyophilized balanced heparin.

In one embodiment, the at least one anticoagulant for the determination of BG and BMP parameters is in lyophilized form when combined with the blood.

In another preferred embodiment, the at least one anti-platelet agent is selected from the group consisting of glycoprotein IIb/IIIa inhibitors, ADP receptors/P2Y₁₂ inhibitors, prostaglandin analogs, COX inhibitors, thromboxane inhibitors, phosphodiesterase inhibitors, cloricromen, ditazole, vorapraxar or combinations thereof.

Glycoprotein Ilb/IIIa, also known as integrin αIIbβ3, is an integrin complex found on platelets. It is a receptor for fibrinogen and von Willebrand factor and aids platelet activation. Glycoprotein IIb/IIIa inhibitors can be used to prevent blood clots in an effort to decrease the risk of heart attack or stroke. Examples of glycoprotein IIb/IIIa include, but are not limited to abciximab, eptifibatide (also called integrillin), orbofiban, lotrafiban, roxifiban, sibrafiban and tirofiban ( also called aggrastat) or a salt thereof. Preferred glycoprotein IIb/IIIa inhibitors are eptifibatide and tirofiban or a salt thereof.

Adenosine diphosphate (ADP) receptor/P2Y₁₂ inhibitors are a drug class of antiplatelet agents, used in the treatment of acute coronary syndrome or as a prevention in patients who are in risk of thromboembolism, myocardial infarction or a stroke. The mechanism of action consists in antagonizing the P2Y₁₂ protein and therefore prevent the binding of ADP to the P2Y₁₂ receptor. This leads to a decrease in aggregation of platelets, prohibiting thrombus formation. The P2Y₁₂ receptor is a surface bound protein found on blood platelets. They belong to G protein-coupled purinergic receptors (GPCR) and are chemoreceptors for ADP. Examples of ADP receptors/P2Y₁₂ inhibitors include, but are not limited to thienopyridines such as clopidogrel, prasugrel and ticlopidine or a salt thereof; and nucleotide/nucleoside analogs/receptor antagonists such as cangrelor, elinogrel, ticagrelor, suramin sodium and 2-MeSAMP. The thienopyridines are less preferred ADP receptors/P2Y₁₂ inhibitors according to the present invention as they are prodrugs that do not show ADP receptors/P2Y₁₂ inhibitory activity in vitro. Consequently, nucleotide/nucleoside analogs/receptor antagonists such as cangrelor, elinogrel, ticagrelor or a salt thereof, suramin sodium and 2-MeSAMP are preferred ADP receptors/P2Y₁₂ inhibitors according to the present invention.

Prostaglandins may induce or inhibit platelet aggregation and constrict to dilate blood vessels. Prostaglandin analogs are a class of drugs that bind to a prostaglandin receptor. Examples include but are not limited to beraprost, iloprost (also known as ZK36374), prostacyclin, epoprostenol, and treprostinil.

Cyclooxygenase (COX), officially known as prostaglandin-endoperoxide synthase (PTGS), is an enzyme that is responsible for formation of prostanoids, including thromboxanes and prostaglandins. Examples of COX inhibitors include, but are not limited to acetlysalicylic acid, aloxiprin, carbasalate calcium, ibuprofen, trifusal, sulfinpyrazone and nitroaspirin (NCX-4016).

Thromboxane is a member of the family of lipids known as eicosanoids. The two major thromboxanes are thromboxane A2 and thromboxane B2. The distinguishing feature of thromboxanes is a 6-membered ether-containing ring. Thromboxane is named for its role in clot formation. Thromboxane-A synthase, an enzyme found in platelets, converts the arachidonic acid derivative prostaglandin H₂ to thromboxane. Thromboxane inhibitors comprise thromboxane synthase inhibitors such as dtritriipyridamole, picotamide, terbogrel, daltroban, seratrodast, SQ-29548 and ramatroban; and thromboxane receptor antagonists such as terbogrel and terutroban.

A phosphodiesterase is an enzyme that breaks a phosphodiester bond. Phosphodiesterase enzymes (PDE) are often targets for pharmacological inhibition due to their unique tissue distribution, structural properties, and functional properties. Inhibitors of phosphodiesterases can prolong or enhance the effects of physiological processes mediated by cAMP or cGMP by inhibition of their degradation by phosphodiesterases. PDE inhibitors have been identified as new potential therapeutics in areas such as pulmonary arterial hypertension, coronary heart disease, dementia, depression, asthma, COPD, protozoal infections, including malaria, and schizophrenia. Further, cyclic adenosine 3',5'-monophosphate (cAMP) and cyclic guanosine 3',5'-monophosphate (cGMP) are two critical intracellular second messengers provided with strong inhibitory activity on fundamental platelet functions. PDEs, by catalysing the hydrolysis of cAMP and cGMP, limit the intracellular levels of cyclic nucleotides, thus regulating platelet function. The inhibition of PDEs may therefore exert a strong platelet inhibitory effect (Gresele et al. Br. J. Clin. Pharmacol. 2011 Oct;72(4):634-46). Examples of PDEs include, but are not limited to Cilostazol, Dipyridamole, Trifusal, Milrinone, Anagrelide and Theophylline.

Anti-platelet drugs which are not known to belong one of the above groups include, but are not limited to cloricromen, ditazole, vorapraxar and L-Arginine or salts thereof.

Cloricromen is an antiplatelet drug with vasodilating activity that is used in the treatment of thromboembolic disorders.

Ditazole is a non-steroidal anti-inflammatory agent with analgesic and antipyretic activity similar to phenylbutazone. Additionally, ditazole is a platelet aggregation inhibitor marketed in Spain and Portugal with trade name Ageroplas^{®}.

Vorapraxar, formerly known as SCH 530348, is a thrombin receptor (protease-activated receptor, PAR-1) antagonist based on the natural product himbacine.

It has been shown that oral L-Arginine inhibits platelet aggregation by way of the nitric oxide pathway (Adams et al., J. Am. Coll. Cardiol. 1995 Oct;26(4): 1054-61).

It is preferred that the at least one anti-platelet agent is selected from the group consisting of glycoprotein IIb/IIIa inhibitors, ADP receptors/P2Y₁₂ inhibitors, prostaglandin analogs, cloricromen, ditazole, vorapraxar or combinations thereof.

In another preferred embodiment, the at least one anti-platelet agent is selected from the group consisting of glycoprotein IIb/IIIa inhibitors, prostaglandin analogs, cloricromen, ditazole, vorapraxar or combinations thereof.

In another preferred embodiment, the at least one anti-platelet agent is selected from the group consisting of glycoprotein IIb/IIIa inhibitors and prostaglandin analogs.

In a preferred embodiment, the at least one antiplatelet agent comprises a prostaglandin analog.

In yet another preferred embodiment, the at least one anti-platelet agent is
a glycoprotein IIb/IIIa inhibitor selected from the group consisting of abciximab, eptifibatide, orbofiban, lotrafiban, roxifiban, sibrafiban and tirofiban or a salt thereof. In a preferred embodiment, the at least one anti-platelet agent comprises eptifibatide and/or tirofiban or a salt thereof. In a preferred embodiment eptifibatide is used as the eptifibatide acetate salt. It is also preferred that tirofiban is used as the tirofiban hydrochloride salt and more preferably the tirofiban hydrochloride monohydrate salt.

In yet another preferred embodiment, the at least one anti-platelet agent is a prostaglandin analog selected from the group consisting of beraprost, iloprost, prostacyclin, epoprostenol, treprostinil or a salt thereof.

In a particularly preferred embodiment, the at least one antiplatelet agent comprises eptifibatide, tirofiban, iloprost, a salt thereof or a combination thereof.

It is especially preferred that the at least one antiplatelet agent comprises iloprost. Iloprost is particularly preferred as the antiplatelet agent as it is not only suitable for the preparing of a blood sample for BG, BMP and platelet count but it was also surprisingly found that it inhibits leukocyte activation and thus a very low WBC aggregation in the blood sample.

In another embodiment, at least one antiplatelet agent comprises ADP receptors/P2Y₁₂ inhibitors, wherein the ADP receptors/P2Y₁₂ inhibitors is a nucleotide/nucleoside analog/receptor antagonist. It is preferred that the ADP receptors/P2Y₁₂ inhibitors is selected from the group consisting of cangrelor, elinogrel, ticagrelor or a salt thereof, suramin sodium, 2-MeSAMP.

In another preferred embodiment, the inventive method does not comprise the step of combining MgSO₄, EDTA or citrate with the blood of the blood sample. These anticoagulant agents are not suitable for preparing a blood sample for BG and BMP parameter analysis.

In another preferred embodiment, the inventive method further comprises the step of mixing the blood sample. The mixing of the blood sample is advantageous as the blood sample may otherwise coagulate or settle or the sample may react with air in the blood samples prior to analysis. The mixing may for example occur by stirring. The mixing may be performed manually by repeatedly inverting the blood sample or by rolling it horizontally. A stirring element may also be included to the blood sample. The sample may then for example be stirred by using moving means, e.g. as described in US 6,880,384 B2. The mixing of the blood sample facilitates the dissolution of anticoagulant, e.g. heparin, and it prevents settling. If the anticoagulant is not properly dissolved, it may lead to formation of micro clots, which may bias the results and/or damage the analyzer. Settling may lead to sample inhomogeneity and misleading analytical results.

In another aspect, the present invention also relates to a blood sampler containing
a) at least one anti-coagulant agent for BG and BMP parameter analysis as described above; and
b) at least one anti-platelet agent as described above.

The blood sampler may be used for performing the inventive method described above. The at least one anti-coagulant agent for BG and BMP parameter analysis may be present in a liquid form within the blood sampler or it may be present in a dry formulation.

In one embodiment, the blood sampler contains a further element comprising the at least one anticoagulant for the determination of BG and BMP parameters and/or at least one anti-platelet agent. For example, the further element may be a "brick" as it is known for "heparin bricks". A "brick" in this context means that the at least one anticoagulant was prepared in a "puff" of inert filler material, wherein the puff dissolves and the heparin is dispersed throughout the sample with proper mixing and wherein the puff could be dispensed during production to deliver a reproducible amount of heparin in each sampler. One example of such a brick is e.g. a piece of cellulose soaked with at least one anticoagulant for the determination of BG and BMP parameters and/or at least one anti-platelet agent. However, also other further elements that may release the at least one anticoagulant for the determination of BG and BMP parameters and/or at least one anti-platelet agent upon contact with blood are possible, e.g. a blood sampler wall coating matrix sprayed on internal sampler surface.

In one embodiment, the blood sampler is comprised of plastic or glass.

In another preferred embodiment, the blood sampler comprises a sampler cap. A sampler cap is a cap to be connected to the open end of a blood sampler, e.g. to the tip of a syringe or to the open end of a capillary tube or a test tube. Gas exchange with the surroundings which may bias the BG analysis results may be avoided by using a sampler cap. Suitable sampler caps are for example described in WO 2004/000412.

In another preferred embodiment, the blood sampler contains a stirring element. It is preferred that the stirring element is a spherical element or a cylindrical element with rounded ends. It is particularly preferred that the stirring element has the form of a ball. The ball may for example be made of steel or plastics.

In another embodiment, the stirring element comprises a coating with an inert material. The inert material does preferably not interfere or not substantially interfere with the blood analysis. For example, the inert material may be selected from the group consisting of gold, platinum, palladium or rhodium. In a preferred embodiment, the inert material is gold.

It is a particularly preferred embodiment that the stirring element is a gold coated ball, preferably a gold coated steel ball.

In one embodiment, the stirring element is a stirring element as described in US 6,880,384 B2.

The movement of the stirring element is also described in US 6,880,384 B2. It is thus preferred that the stirring element is moved by moving means, e.g. by mechanical means. The moving means may be a robot arm or a support for moving, e.g. tilting or rotating, the sample handler and/or the sampler bed, thereby moving the stirring element held in any sampler therein by means of gravitational forces.

In a preferred embodiment, the blood sampler may be filled with a blood volume of 20 µL to about 10 mL, preferably from about 35 µL to about 10 mL, more preferably from 50 µL to about 5 mL and even more preferably from about 0.5 to about 2 mL blood.

In another aspect, the present invention relates to the use of at least one anticoagulant for the determination of blood gas and BMP parameters and at least one anti-platelet agent for the preparation of a blood sample for blood gas and BMP analysis and platelet count. The at least one anti-platelet agent and the at least one anticoagulant for the determination of BG and BMP parameters are as described above. In a preferred embodiment, the blood sample is prepared in a blood sampler.

In yet another aspect, the present invention relates to the use of a blood sampler comprising at least one anticoagulant for the determination of blood gas and BMP parameter analysis and at least one anti-platelet agent for the analysis of blood gas, BMP parameter and/or platelet count. The blood sampler, the at least one anti-platelet agent and the at least one anticoagulant for the determination of BG and BMP parameters are as described above. In a preferred embodiment, the blood sampler further contains blood.

As used herein, the term "parameter" means any piece of clinical information about the blood sample.

As used herein, the term "blood gas" as in "blood gas parameter" refers to gaseous parameters of the blood and includes the amounts of certain gases (e.g. oxygen and carbon dioxide) dissolved in blood, typically arterial blood. Blood gas parameters include the pH, pCO₂, pO₂, oxygen saturation (sO₂), the concentration of total hemoglobin (ctHb or tHb), the fraction of oxyhemoglobin (FO₂Hb or O₂Hb), the fraction of carboxyhemoglobin (FCOHb or COHb), the fraction of methemoglobin (FMetHb or MetHb), the fraction of deoxyhemoglobin (FHHb or RHb), and the fraction of fetalhemoglobin (FHbF). A "blood sample suitable for blood gas analysis" means that the blood sample may be used for measuring at least one blood gas parameter but is preferably suitable to measure all blood gas parameters of pH, pCO₂, pO₂, ctHb, FO₂Hb, FCOHb, FMetHb, FHHb, and FHbF. It may be preferred that at least pH, tHb, FCOHb, and FMetHb can be measured.

As used herein, the term "basic metabolic panel" as in "basic metabolic panel parameter analysis" refers to biochemical blood parameters, in particular electrolytes, namely the concentration of Na⁺, K⁺, Cl⁻, HCO₃⁻, urea, creatinine, glucose (glu), Ca²⁺, lactate (lac) and total bilirubin (tBil). A "blood sample suitable for BMP parameter analysis" is thus a blood sample that can be used to determine at least one but preferably all of the above BMP parameters. It may be preferred that at least Na⁺, K⁺, Cl⁻, Ca²⁺, glu, lac and tBil can be measured.

As used herein, the term "platelet count" or "determining the number of platelets" means a diagnostic test that determines the number of platelets in the patient's blood. Platelets, which are also called thrombocytes, are small disk-shaped blood cells produced in the bone marrow and involved in the process of blood clotting. There are normally between 150,000-450,000 platelets in each microliter of blood. Low platelet counts or abnormally shaped platelets are associated with bleeding disorders. High platelet counts sometimes indicate disorders of the bone marrow.

As used herein, the term "at least one" as in "at least one anticoagulant" or "at least one anti-platelet agent" means that only one type of agent or different agents, e.g. different anticoagulants, may be present in the sample. In a preferred embodiment, "at least one" means "one" type of agent, e.g. one type of anticoagulant, e.g. heparin.

As used herein, the term "anticoagulant" means a substance that prevents or reduces the coagulation of blood, i.e. the coagulation cascade leading to fibrin polymerization and therefore fibrin clot formation. Anticoagulants thereby prolong the clotting time by inhibiting the coagulation cascade by clotting factors after the initial platelet aggregation.

As used herein, the term "anticoagulant for the determination of BG and BMP parameters" means that a substance is suitable as anticoagulant in a blood sample so that the blood sample can be used for the analysis of BG and BMP parameters. Some anticoagulants, e.g. EDTA, are known to be unsuitable for the determination of BG and BMP parameters, e.g. because they form a complex with Ca²⁺ so that the calcium concentration cannot be reliably determined in a blood sample. Thus, EDTA is not an anticoagulant for the determination of BG and BMP parameters.

As used herein, the term "anti-platelet agent" means a substance that decreases platelet aggregation and/or inhibits thrombus formation, i.e. a substance that inhibits the initial platelet aggregation of the blood clotting. Anti-platelet agents thus interfere with the platelet activation cascade leading to activated platelets which can adhere to fibrin fibers, other extracellular matrix components or aggregate into platelet aggregates. It is emphasized that the coagulation cascade and the platelet aggregation cascade are two separate cascades, even though some proteins, such as thrombin, may play a role in both cascades. Antiplatelet drugs can reversibly or irreversibly inhibit the process involved in platelet activation resulting in decreased tendency of platelets to adhere to one another and to damaged blood vessels endothelium or to foreign material surfaces, as e.g. a blood sampler material.

As used herein, the term "blood sample" or "blood analysis sample" refers to a sample of blood that is suitable for diagnostic or analytical purposes. Thus, the blood sample comprises a relatively low volume of blood (from 20 µL to 10 mL blood), i.e. not the volumes e.g. required for blood donations (up to about 450 mL blood). A "blood sample suitable for BG and BMP parameter analysis and platelet count" means that the blood sample is suitable for use in the determination of BG, BMP parameters as well as performing a platelet count, wherein the anticoagulant and/or the anti-platelet agent does not interfere or at least not substantially interfere with the determination of one of the parameters.

As used herein, the term "blood sampler" means a device for collection of blood, such as a syringe, a capillary tube, or a test tube, e.g. an aspirating sampler or self-aspirating sampler, such as a PICO syringe (Radimeter Medical ApS), a vacuum test tube or a similar device designated for blood sampling.

As used herein, the term "white blood cell count" means a diagnostic test counting the number of leukocytes in a sample of the patient's blood. An average normal range is between 3,500 and 10,500 white blood cells per µL blood.

As used herein, the term "complete cell count" means a diagnostic test including a white blood cell count, platelet count and either a 3-diff (3-part diff) or a 5-diff (5-part diff). The complete cell count does not include a count of red blood cells of the sample of the patient's blood.

As used herein, "not substantially interfere" means that the fraction of maximum interference is less than 5, preferably less than 2.5 and more preferably 1 or less.

As used herein, the term "about" in the context of numeric values in the context of the present application, as e.g. in "about 10 mL", means that the value recited immediately after the "about" also comprises minor deviations from the exact numeric value, e.g. due to measuring errors etc. In a preferred embodiment, the term "about" means a value within 15% (±15%) of the value recited immediately after the term "about," including any numeric value within this range, the value equal to the upper limit (i.e. +15%) and the value equal to the lower limit (i.e. -15%) of this range. For example, the phrase "about 100" encompasses any numeric value that is between 85 and 115, including 85 and 115 (with the exception of "about 100%", which always has an upper limit of 100%). In one aspect, "about" means ±10%, even more preferably ±5%, even more preferably ±1% or less than ±1%.

In general, the present invention preferably includes all salts of the disclosed reagents such as the at least one anti-platelet agent or the at least one anticoagulant for the determination of BG and BMP parameters, provided that these salts do not interfere or not substantially interfere with the blood analysis. Examples of salts include inorganic and organic acid addition salts and basic salts. The salts include, but are not limited to, metal salts such as cesium salt, alkaline salts such as lithium salt, sodium salt, potassium salt, calcium salt or magnesium salt, organic amine salts such as triethylamine salt, pyridine salt, picoline salt, ethanolamine salt, triethanolamine salt, dicyclohexylamine salt, N,N'dibenzylethylenediamine salt and the like; inorganic acid salts such as citrate, tartrate, maleate, fumarate, mandelate, acetate, dichloroacetate, trifluoroacetate, dicloroacetate, trifluoroacetate, oxalate, formate and the like, sulfonates such as methanesulfonate, benzenesulfonate, p-toluenesulfonate and the like; and amino acid salts such as arginate, glutamate and the like. Acid addition salts include, but are not limited to, hydrochloric acid, fumaric acid, maleic acid, succinic acid, acetic acid, citric acid tartaric acid, phosphoric acid, oxalic acid, dichloroacetic acid and the like.

### Brief description of Figures

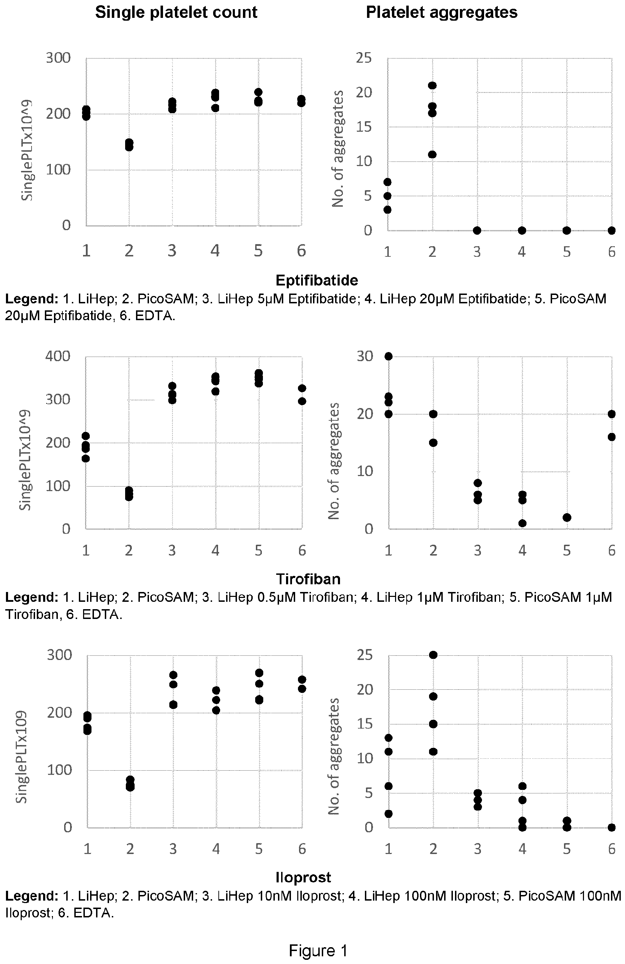

Figure 1: Single platelet counts and number of platelet aggregates quantified in blood collected with PICO70 sampler containing only liquid heparin (LiHep, no gold-coated (Au) ball, gentle manual mixing), PICO70 mixed on SAM mixer (PicoSAM), EDTA anticoagulant and liquid heparin or full PicoSAM in combination with two concentration levels of anti-platelet drugs eptifibatide, tirofiban or iloprost. The three anti-platelet drugs show single platelet counts and no or few aggregates comparable to standard EDTA anticoagulated blood.

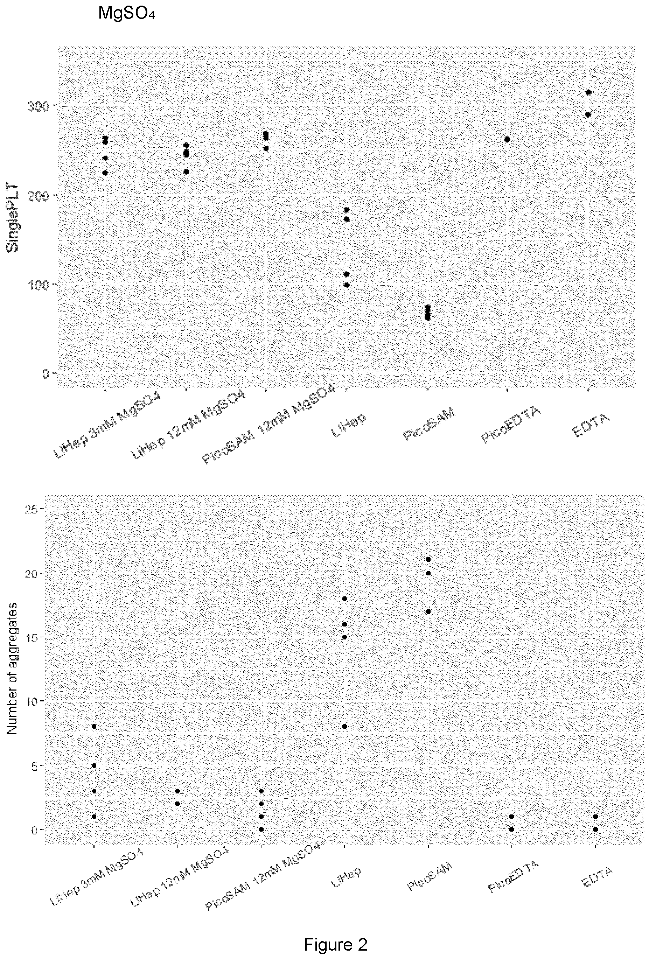

Figure 2: Single platelet counts and number of platelet aggregates quantified in blood collected with PICO70 sampler containing only liquid heparin (LiHep, no Au ball, gentle manual mixing), PICO70 mixed on SAM mixer (PicoSAM), EDTA anticoagulant and liquid heparin or full PicoSAM in combination with two concentration levels of anti-platelet drug MgSO₄.

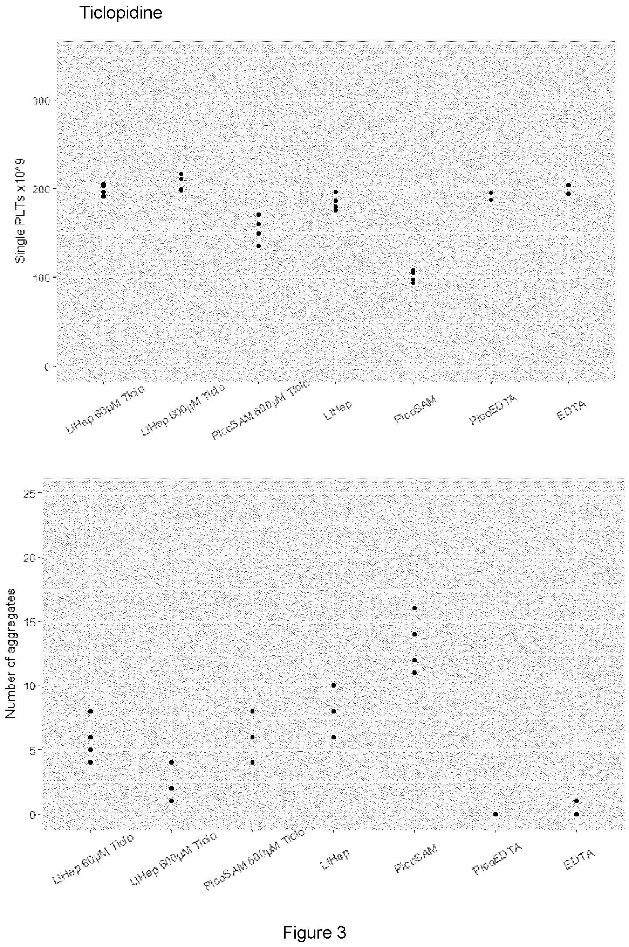

Figure 3: Single platelet counts and number of platelet aggregates quantified in blood collected with PICO70 sampler containing only liquid heparin (LiHep, no Au ball, gentle manual mixing), PICO70 mixed on SAM mixer (PicoSAM), EDTA anticoagulant and liquid heparin or full PicoSAM in combination with two concentration levels of anti-platelet drug ticlopidine.

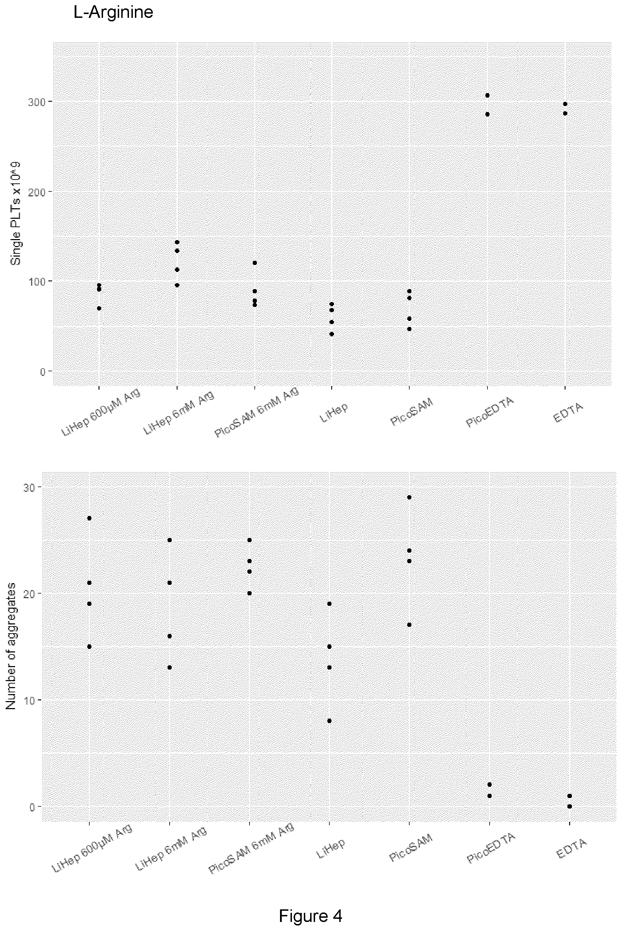

Figure 4: Single platelet counts and number of platelet aggregates quantified in blood collected with PICO70 sampler containing only liquid heparin (LiHep, no Au ball, gentle manual mixing), PICO70 mixed on SAM mixer (PicoSAM), EDTA anticoagulant and liquid heparin or full PicoSAM in combination with two concentration levels of anti-platelet drug L-Arginine.

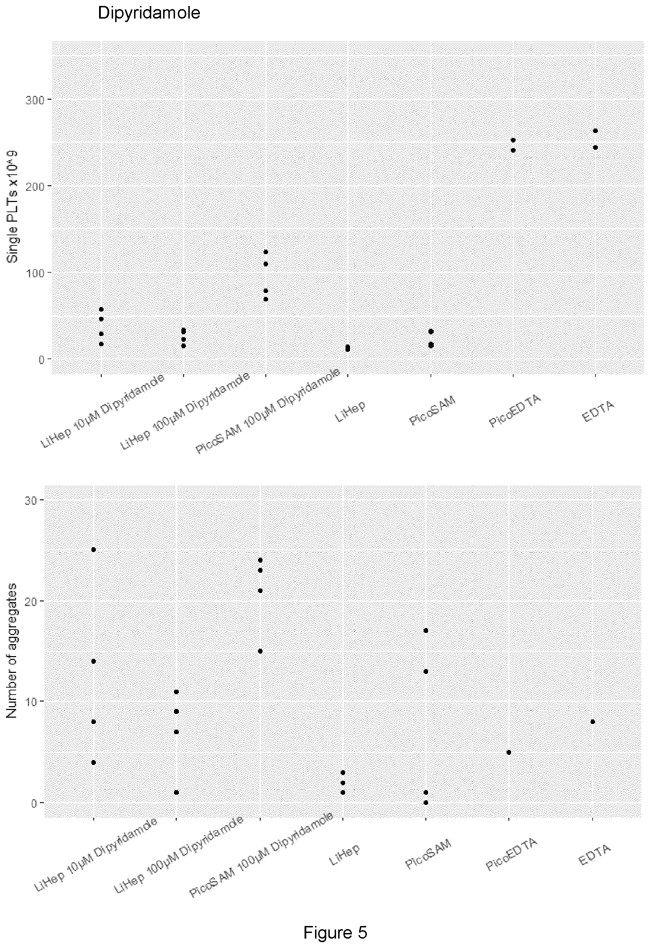

Figure 5: Single platelet counts and number of platelet aggregates quantified in blood collected with PICO70 sampler containing only liquid heparin (LiHep, no Au ball, gentle manual mixing), PICO70 mixed on SAM mixer (PicoSAM), EDTA anticoagulant and liquid heparin or full PicoSAM in combination with two concentration levels of anti-platelet drug dipyridamole.

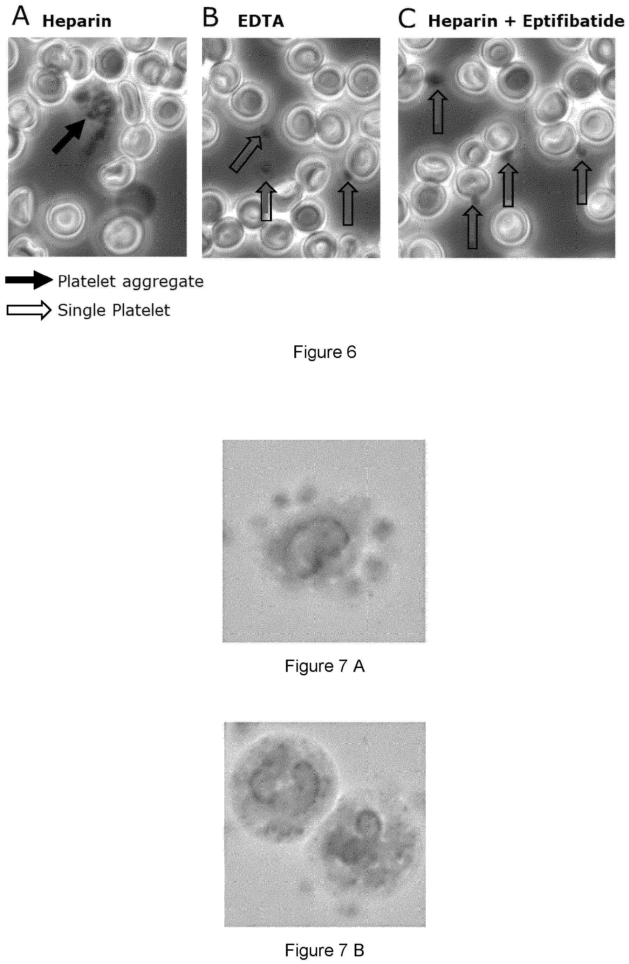

Figure 6: Microscopic images of blood samples anticoagulated with (A) heparin and (B) EDTA. While EDTA prevents platelet activation and maintains single platelets in the blood sample, heparin allows or even potentiates platelet aggregation induced by other agonists or foreign materials. This effect of heparin cannot be observed in microscopic images when (C) 20 µM eptifibatide is added to the heparin.
Figure 7: Blood sample handmixed with staining/hemolyzing reagent. Stained WBCs (white blood cells) and platelets prepared in a wet mount on a glass coverslip were imaged with a Leica microscope using a 40x objective in bright field mode. (A) Example of white blood cells showing interaction with platelets. (B) Example of white blood cell aggregates.

### Examples

### Example 1 Analysis of platelet aggregation using heparin and different anti-platelet agents

Each experiment was conducted on a separate day with blood from one voluntary donor testing one anti-platelet drug candidate. For each experiment PICO70 syringe samplers (Radiometer Medical ApS) were prepared just before sample drawing. For "LiHep" (Liquid Heparin) conditions, PICO70 samplers were emptied of the gold ball and heparin brick and 15µl of aqueous liquid balanced heparin comprising heparin lithium (Celsus Laboratories) and heparin sodium (Celsus Laboratories) (final heparin conc. 60IU/mL blood) and 15µl of the solvent of the tested drug were added. For "PicoSAM" conditions, unmodified PICO70 samplers (with gold ball and heparin brick) were used and 15µl of the solvent of the respective tested drug was added. For "LiHep xxx drug" conditions, PICO70 samplers were emptied as described for LiHep and 15µl of liquid balanced heparin (final heparin conc. 60IU/mL blood) and 15µl of the dissolved tested drug were added. For "PicoSAM xxx drug" conditions, unmodified PICO70 samplers (with gold ball and heparin brick) were used and 15µl of the dissolved tested drug was added. The tested anti-platelet drug candidates and the corresponding solvents were eptifibatide acetate (Sigma, SML1042; dissolved in saline, final conc. 5 and 20µM), MgSO₄ (Sigma, M7506; dissolved in saline, final conc. 3 and 12mM), tirofiban hydrochloride monohydrate (Sigma, SML0246; dissolved in 1:200 DMSO in saline, final conc. 0.5 and 1µM), iloprost (Sigma, SML1651; dissolved in 1:1000 or 1:10000 ethanol in saline, final conc. 10 and 100nM (later also 1µM)), ticlopidine hydrochloride (dissolved in saline, final conc. 60 and 600µM), L-Arginine (Sigma, A5006; dissolved in saline, final conc. 600µM and 6mM) and dipyridamole (Sigma, D9766; dissolved in 1:10 or 1:100 DMSO in saline, final conc. 10 and 100µM). An EDTA tube (BD Vacutainer with spray-coated K₂EDTA, 10ml) and duplicate samples of all conditions of PICO70 syringe samplers were filled by drawing venous blood via a butterfly needle using a sealed VTC (vented tip cap) to fill the otherwise self-aspirating PICO70 samplers with 1.5 mL venous whole blood. Samplers were inverted 8 times immediately after drawing to insure proper anticoagulation of the samples. The blood samples were mixed gently by hand (samplers without gold ball) or on a SAM mixer (Radiometer Medical ApS) for 15 minutes after drawing the sample. Mixed samples were fixed immediately with 10% formalin solution (1:1 dilution of blood in formalin) for at least 10minutes and further diluted 1:10 in platelet dilution solution to hemolyze red blood cells (RBCs) to assessed manual platelet count using a hemocytometer. Manual platelet counts of single platelets (not aggregated), number of platelet aggregates and if possible size (number of platelets in an aggregate) of platelet aggregates were quantified in duplicate on each sample by counting platelets in a hemocytometer using a Leica 750 microscope with 10× and 20x phase-contrast air objectives. Single platelet counts were adjusted for dilution with liquid heparin and or dissolved drug solution to calculate single platelet concentrations.

Eptifibatide, tirofiban and iloprost reduced the formation of platelet aggregates compared to "LiHep" and "PicoSAM" references (see Figure 1).

MgSO₄ also reduced the formation of platelet aggregates (see Figure 2).

Ticlopidine LiHep showed a similar count of single platelets compared to EDTA reference and a slightly reduced number of aggregates compared to the LiHep and PicoSAM controls (see Figure 3). L-Arginine showed a decreased number of platelets and a higher number of platelet aggregates compared to EDTA control but still a slightly higher platelet count than the LiHep and PicoSAM controls (see Figure 4).

Dipyridamole, a phosphodiesterase inhibitor and thromboxane inhibitor, showed a slightly higher platelet number compared to the LiHep and PicoSAM controls (see Figure 5).

### Example 2 Platelet aggregation studies with heparinized blood and EDTA-treated blood

Venous blood samples drawn, mixed and fixed with 10% formalin solution, as described before in Example 1 were used to prepare wet mounts on a glass slide and covered with a glass coverslip. Wet mount samples of fixed blood cells were then imaged with a Leica 750 microscope using a 40x phase-contrast air objective. Aggregated platelets in heparinized blood versus non-aggregated single platelets in EDTA anticoagulated blood and heparinized blood with e.g. eptifibatide are seen between abundant RBCs.

The results are shown in Figure 6. Heparinized blood showed platelet aggregates which cannot be seen in blood samples prepared with EDTA or in a blood sample prepared with heparin and an antiplatelet drug such as eptifibatide.

### Example 3 Wet mount images of stained blood samples

Venous blood samples were drawn and mixed as described before in Example 1 and used to prepare stained wet mounts images. Blood samples were mixed with a staining and hemolyzing agent (methylene blue and deoxycholic acid, respectively) and incubated at 47°C in a water bath for 30 seconds. Stained and hemolyzed blood samples were then prepared in wet mount samples on a glass slide and covered with a glass coverslip. Images of stained samples were then acquired with a Leica 750 microscope using a 40x air objective in bright field mode. An image processing software (FIJI, ImageJ) was used to select regions of interest (ROIs) of representative stained WBCs from the images.

PicoSAM samples (heparinized blood without an anti-platelet drug) showed many aggregated platelets (small roundish cells).

PicoSAM samples with 1 µM tirofiban or 20 µM eptifibatide showed single platelets but also platelet satellinism, i.e. platelets binding to WBCs. This is for example shown in Figure 7A. Further, WBC aggregates were also observed as shown in Figure 7B.

PicoSAM samples with 100 nM iloprost (or samples with balanced LiHep and 100 nM Iloprost) showed single platelets, not platelet satellinism or WBC aggregates. The same results were obtained using EDTA anticoagulated blood.

### Example 4 Single platelet concentrations of Iloprost heparin samples compared to EDTA reference samples

### Manual platelet count

Samples were prepared as described in Example 1.

### ABX platelet count

Blood samples drawn and mixed after 15minutes as described above were assessed with an automated hematology analyzer (Horiba, ABX Pentra 60C+) (ABX) for complete blood count (CBC) with 5-diff including WBC concentration, platelet concentration, mean platelet volume (MPV), concentrations and fractions of neutrophils, lymphocytes, monocytes, eosinophils and basophils, RBC concentration, hematocrit, hemoglobin concentration, RBC descriptive parameters (MCV, MCH, MCHC, RDW). Samples were prepared from several donors. Measured platelet concentration was used to compare to manually assessed platelet concentrations of iloprost heparin samples (in PICO70 samplers) and EDTA anticoagulated samples. The ABX analyzer can underestimate platelet concentrations if platelets are aggregated, though through shear forces in the flow system weakly aggregated platelets might to a certain extent be disaggregated. For control, EDTA samples with no platelet aggregates are used as reference measurement for the measured automated ABX platelet concentration.

For one donor, the average performance of platelet count of PicoSAM 100 nM iloprost samples compared to EDTA samples as reference was 97% for the manual count.

For eight donors, the average performance of platelet count of PicoSAM 1 µM iloprost samples compared to EDTA samples as reference was 93% for the manual count.

The respective PicoSAM sample, i.e. without iloprost but with heparin, in contrast showed a performance of less than 40% compared to the EDTA reference.

For seven donors, the average performance of platelet count of PicoSAM 1 µM iloprost samples compared to EDTA samples as reference was 97% for the ABX platelet count.

Table 1 shows the results of platelet counts for donors samples where both types of platelet counts (manual and ABX platelet count) were performed.

**Table 1. Comparison platelet count**

| Donor ID | | **Mean ABX PLT count (PLT/nl)** | **Mean Manual PLT count (PLT/nl)** |
|---|---|---|---|
| 4 | **PicoSAM Iloprost 1µM** | **204** | **198** |
| | EDTA | 200 | 200 |
| 5 | **PicoSAM Iloprost 1µM** | **253** | **242** |
| | EDTA | 259 | 242 |
| 6 | **PicoSAM Iloprost 1µM** | **173** | **154** |
| | EDTA | 185 | 189 |
| 7 | **PicoSAM Iloprost 1µM** | **233** | **247** |
| | EDTA | 240 | 249 |
| 8 | **PicoSAM Iloprost 1µM** | **191** | **203** |
| | EDTA | 202 | 207 |
| 9 | **PicoSAM Iloprost 1µM** | **216** | **202** |
| | EDTA | 236 | 255 |

### Example 5 Anti-platelet drug interference test on ABL90 parameters

Each experiment was conducted on a separate day with blood from one voluntary donor. For each experiment three PICO70 syringe samplers (Radiometer Medical ApS) (not modified, containing a gold ball and a heparin brick) were prepared. One PICO70 was used unmodified (Ctrl), one PICO70 was filled with 15µl of dissolved anti-platelet drug to reach the indicated final concentration in the blood sample. A third PICO70 sampler was filled with 15µl of solvent (specific solvent, which is used to dissolve the tested anti-platelet drug: reference sample). Venous blood samples were drawn into PICO70 syringe samplers via a butterfly needle and a sealed vented tip cap (VTC) to fill the self-aspirating PICO70 samplers with 1.5ml whole blood. Samplers were inverted eight times immediately after drawing to insure proper heparinization of the sample. Theblood samples were mixed on a SAM mixer (Radiometer Medical ApS), moving the gold-coated steel ball through the blood sample in order to ensure homogenous and reproducible mixing for 15 minutes after drawing of the blood, and analyzed on an ABL90 blood gas analyzer (Radiometer Medical ApS). Samples were measured in alternating order (Ctrl, Reference sample, Drug sample) five times each (5 replicate measurements) with gentle manual inversion of the samples in order to keep blood samples homogenously mixed through the measurements. All calculated values are described in Table 3. Afterwards blood samples were centrifuged to separate the plasma fraction. Plasma of all samples was measured in triplicate in alternating order on the ABL90 instrument to assess the free Hemoglobin concentration in the plasma, which indicates a possible problematic hemolyzation of red blood cells (RBCs) in the whole blood sample. Hemolyzed samples show interference on e.g. the measured K⁺ concentration.

Measured parameters are described in Table 2.

**Table 2. Parameters measured with ABL90 blood gas analyzer**

| **Parameter** | **Description** |
|---|---|
| pH | pH |
| tHb (g/dL) | total Hemoglobin concentration |
| COHb (%) | Carboxyhemoglobin (carboxylated Hemoglobin) |
| MetHb (%) | Methemoglobin (methylated Hemoglobin) |
| K⁺ (mmol/L) | Potassium concentration |
| Na⁺ (mmol/L) | Sodium concentration |
| Ca²⁺ (mmol/L) | Calcium concentration, ionized |
| Cl⁻ (mmol/L) | Chloride concentration |
| Glu (mmol/L) | Glucose concentration |
| Lac (mmol/L) | Lactate concentration |
| tBil (µmol/L) | Bilirubin concentration |

The nomenclature of how the calculations have been made is described in Table 3 and the nomenclature of the measured samples is described in Table 4.

**Table 3. Calculated values**

| | |
|---|---|
| Mean | Mean value of 5 replicate measurements performed on the same whole blood sample or 3 replicate measurements of Hemoglobin measured on plasma sample (data not shown) |
| SD | Standard deviation calculated from 5 replicate measurements (data not shown) |
| Difference | Difference in measured parameter between indicated samples (test sample with drug - reference sample with solvent only) (data not shown) |
| Fraction of maximum interference | Difference divided by maximum allowed interference for each parameter (a value >1 indicates an interference, whereas <1 are acceptable) |

**Table 4. Sample names**

| | |
|---|---|
| Ctrl | Control sample drawn into unmodified PICO70 syringe sampler |
| Saline | Reference sample containing solvent saline (physiological NaCl solution) |
| DMSO/Saline (1:200) | Reference sample containing solvent DMSO in saline (1:200 dissolved) |
| EtOH/Saline (1:1000) | Reference sample containing solvent ethanol (EtOH) in saline (1:1000 dissolved) |
| EtOH/Saline (1:100) | Reference sample containing solvent ethanol (EtOH) in saline (1:100 dissolved) |
| Eptifibatide 20uM in saline | Eptifibatide dissolved in saline to reach final conc. of 20µM eptifibatide in the blood sample |
| Tirofiban 1uM in DMSO/Saline | Tirofiban dissolved inDMSO/saline solvent (1:200) to reach final conc. of 1µM tirofiban in the blood sample |
| Iloprost 100nM in EtOH/Saline | Iloprost dissolved in ethanol/saline solvent (1:1000) to reach final conc. of 100nM iloprost in the blood sample |
| Iloprost 1uM in EtOH/Saline | Iloprost dissolved in ethanol/saline solvent (1:100) to reach final conc. of 1µM iloprost in the blood sample |
| MgSO₄ 12mM in saline | MgSO₄ dissolved in saline to reach final conc. of 12mM MgSO₄ in the blood sample |

Reference range for adults and maximum interference allowed in the examples are shown in Table 5.

The results of the measurements (as fraction of maximum interference) are summarized in Table 6.

**Table 5. Reference values for determining maximum allowed interference.**

| | **pH** | **tHb (g/dL)** | **COHb (%)** | **MetHb (%)** | **K⁺ (mmol/L)** | **Na⁺ (mmol/L)** |
|---|---|---|---|---|---|---|
| **Reference range adult** | 7.32-7.43 | 11.4-17.5 | 2-3% (non-smokers) | 1-2% | 3.4-4.5 | 136-145 |
| | | | 7-9% (smokers) | | | |
| **max. Interference allowed** | <0.010 | <0.5 | <1% | <1% | <0.1 | <1 |

| | **Ca²⁺ (mmol/L)** | **Cl-(mmol/L)** | **Glu (mmol/L)** | **Lac (mmol/L)** | **tBil (µmol/L)** | |
|---|---|---|---|---|---|---|
| **Reference range adult** | 1.15-1.33 | 98-107 | 3.6-5.3 | 0.56-1.39 | 0-34 | |
| **max. Interference allowed** | 0.02 | 1 | 0.1 | 0.1 | 30 | |

**Table 6. Fraction of maximum interference of parameter for antiplatelet drugs**

| | **Eptifibatide** | **Tirofiban** | **Iloprost** | **MgSO₄** |
|---|---|---|---|---|
| **pH** | 0.18 | 0.18 | 0.32 | *5.56* |
| **tHb (g/dL)** | 0.04 | 0.12 | 0.12 | 0.08 |
| **COHb (%)** | 0.02 | 0.06 | 0.08 | 0.04 |
| **MetHb (%)** | 0.08 | 0.04 | 0.04 | 0.04 |
| **K⁺ (mmol/L)** | 0 | 0 | 0.18 | *2.0* |
| **Na⁺ (mmol/L)** | 0.4 | 0 | 0.14 | *5.6* |
| **Ca²⁺ (mmol/L)** | 0.2 | 0.1 | 0.1 | *2.7* |
| **Cl⁻ (mmol/L)** | 0 | 0 | 0.06 | *8.6* |
| **Glu (mmol/L)** | 0 | 0.8 | 0.86 | 0.2 |
| **Lac (mmol/L)** | 0.6 | 0 | 0.62 | *4.6* |
| **tBil (µmol/L)** | 0.013 | 0.000 | 0.013 | 0.140 |

Eptifibatide, tirofiban and iloprost showed no interference on assessed ABL parameters, whereas MgSO₄ shows interference (Difference Fraction max. Interf. >2) on multiple parameters (pH, N⁺, K⁺, Ca²⁺, Cl⁻, and Lac).

Eptifibatide, tirofiban and iloprost should therefore be safe to add to heparinized blood used for measurement of blood gas and basic metabolic panel parameters at the tested concentrations.

### Aspects

Aspect 1. An in vitro method for preparing a blood sample suitable for blood gas and basic metabolic panel (BMP) parameter analysis and platelet count, wherein blood is combined with
   a. at least one anticoagulant for the determination of blood gas and basic metabolic panel parameters; and
   b. at least one anti-platelet agent.
Aspect 2. A method for preparing a blood analysis sample comprising the step of
   a. drawing blood from a patient into a blood sampler;
   wherein the blood sampler contains at least one anticoagulant for the determination of blood gas and BMP parameter analysis and at least one anti-platelet agent.
Aspect 3. An in vitro method for determining blood gas and BMP parameter and platelet count in a blood sample comprising the step of
   a. determining blood gas, BMP parameter and/or number of platelets in a blood sample obtained by the method of aspect 1 or 2.
Aspect 4. The method of any one of aspects 1 to 3, wherein the at least one anticoagulant is heparin.
Aspect 5. The method of any one of aspects 1 to 4, wherein the at least one anti-platelet agent is selected from the group consisting of glycoprotein IIb/IIIa inhibitors, ADP receptors/P2Y₁₂ inhibitors, prostaglandin analogs, COX inhibitors, thromboxane inhibitors, phosphodiesterase inhibitors, cloricromen, ditazole, vorapraxar or combinations thereof.
Aspect 6. The method of any one of aspects 1 to 5, wherein the at least one anti-platelet agent is selected from the group consisting of glycoprotein IIb/IIIa inhibitors and prostaglandin analogs.
Aspect 7. The method of any one of aspects 1 to 6, wherein the at least one anti-platelet agent is
   a. a Glycoprotein IIb/IIIa inhibitor selected from the group consisting of abciximab, eptifibatide, orbofiban, lotrafiban, roxifiban, sibrafiban and tirofiban or a salt thereof; or
   b. a prostaglandin analog selected from the group consisting of beraprost, iloprost, prostacyclin, epoprostenol, treprostinil or a salt thereof.
Aspect 8. The method of any one of aspects 1 to 7, wherein the at least one antiplatelet agent comprises eptifibatide, tirofiban, iloprost, a salt thereof or a combination thereof.
Aspect 9. The method of any one of aspects 1 to 8, wherein the at least one antiplatelet agent comprises iloprost.
Aspect 10. The method of any one of aspects 1 to 9, wherein the method comprises the further step of mixing the blood sample.
Aspect 11. Use of at least one anticoagulant for the determination of blood gas and BMP parameters and at least one anti-platelet agent for the preparation of a blood sample for blood gas and BMP analysis and platelet count.
Aspect 12. Use of a blood sampler comprising at least one anticoagulant for the determination of blood gas and BMP parameter analysis and at least one anti-platelet agent for the analysis of blood gas, BMP parameter and/or platelet count.
Aspect 13. Blood sampler containing
   a. at least one anti-coagulant agent for blood gas and BMP parameter analysis; and
   b. at least one anti-platelet agent.
Aspect 14. Blood sampler of aspect 13, wherein the least one anti-coagulant agent for blood gas and BMP parameter analysis is heparin and/or the at least one anti-platelet agent is selected from the group consisting of glycoprotein IIb/IIIa inhibitors, ADP receptors/P2Y₁₂ inhibitors, prostaglandin analogs, COX inhibitors, thromboxane inhibitors, phosphodiesterase inhibitors, cloricromen, ditazole, vorapraxar or combinations thereof.
Aspect 15. Blood sampler of aspect 13 or 14, wherein the at least one anti-platelet agent is selected from the group consisting of glycoprotein Ilb/IIIa inhibitors and prostaglandin analogs.
Aspect 16. Blood sampler of any one of aspects 13 to 15, wherein the at least one antiplatelet agent comprises eptifibatide, tirofiban, iloprost, a salt thereof, or a combination thereof.
Aspect 17. Blood sampler of any one of aspects 13 to 16, wherein the at least one antiplatelet agent comprises iloprost.
Aspect 18. Blood sampler of any one of aspects 13 to 17, wherein the blood sampler contains a stirring element.
Aspect 19. Blood sampler of aspect 18, wherein the stirring element is a spherical element or a cylindrical element with rounded ends.
Aspect 20. Blood sampler of any one of aspects 13 to 19, wherein the blood sampler contains at least one further element comprising the at least one anticoagulant for the determination of blood gas and BMP parameters and/or at least one anti-platelet agent.

## Claims

1. An in vitro method for determining blood gas and BMP parameter and platelet count in a blood sample comprising the step of
a) determining blood gas, BMP parameter and number of platelets in a blood sample obtained by combining blood with
i. at least one anticoagulant for the determination of blood gas and basic metabolic panel parameters; and
ii. at least one anti-platelet agent.

2. The method of claim 1, wherein the at least one anticoagulant is heparin.

3. The method of any one of claims 1 to 2, wherein the at least one anti-platelet agent is selected from the group consisting of glycoprotein IIb/IIIa inhibitors, ADP receptors/P2Y₁₂ inhibitors, prostaglandin analogs, COX inhibitors, thromboxane inhibitors, phosphodiesterase inhibitors, cloricromen, ditazole, vorapraxar or combinations thereof.

4. The method of any one of claims 1 to 3, wherein the at least one anti-platelet agent is selected from the group consisting of glycoprotein Ilb/IIIa inhibitors and prostaglandin analogs.

5. The method of any one of claims 1 to 4, wherein the at least one anti-platelet agent is
i) a Glycoprotein IIb/IIIa inhibitor selected from the group consisting of abciximab, eptifibatide, orbofiban, lotrafiban, roxifiban, sibrafiban and tirofiban or a salt thereof; or
ii) a prostaglandin analog selected from the group consisting of beraprost, iloprost, prostacyclin, epoprostenol, treprostinil or a salt thereof.

6. The method of any one of claims 1 to 5, wherein the at least one antiplatelet agent comprises eptifibatide, tirofiban, iloprost, a salt thereof or a combination thereof.

7. The method of any one of claims 1 to 6, wherein the at least one antiplatelet agent comprises iloprost.

8. The method of any one of claims 1 to 7, wherein the method comprises the further step of mixing the blood sample.

9. Blood sampler containing
a) at least one anti-coagulant agent for blood gas and BMP parameter analysis; and
b) at least one anti-platelet agent.

10. Blood sampler of claim 9, wherein the least one anti-coagulant agent for blood gas and BMP parameter analysis is heparin and/or the at least one anti-platelet agent is selected from the group consisting of glycoprotein Ilb/IIIa inhibitors, ADP receptors/P2Y₁₂ inhibitors, prostaglandin analogs, COX inhibitors, thromboxane inhibitors, phosphodiesterase inhibitors, cloricromen, ditazole, vorapraxar or combinations thereof, preferably, wherein the at least one anti-platelet agent is selected from the group consisting of glycoprotein Ilb/IIIa inhibitors and prostaglandin analogs.

11. Blood sampler of any one of claims 9 to 10, wherein the at least one antiplatelet agent comprises eptifibatide, tirofiban, iloprost, a salt thereof, or a combination thereof.

12. Blood sampler of any one of claims 9 to 11, wherein the at least one antiplatelet agent comprises iloprost.

13. Blood sampler of any one of claims 9 to 12, wherein the blood sampler contains a stirring element.

14. Blood sampler of claim 13, wherein the stirring element is a spherical element or a cylindrical element with rounded ends.

15. Blood sampler of any one of claims 9 to 14, wherein the blood sampler contains at least one further element comprising the at least one anticoagulant for the determination of blood gas and BMP parameters and/or at least one anti-platelet agent.
